# FASCICULE DE BREVET EUROPEEN

(11) **EP 2 568 283 B1**
(45) Date de publication et mention de la délivrance du brevet: **04.11.2015**
(21) Numéro de dépôt: 12181259.8
(22) Date de dépôt: 21.08.2012
(51) Int. Cl.: G01N 33/00, G01N 33/543, G01N 33/66, G01N 30/88

(54) **Procédé de détermination d'une empreinte chimique spécifique de la production de mycotoxines**
Verfahren zur Bestimmung eines spezifischen chemischen Fingerabdrucks der Produktion von Mykotoxinen
Method for determining a specific chemical fingerprint of the production of mycotoxins

(30) Priorité: 08.09.2011 FR 1157992
(43) Date de publication de la demande: 13.03.2013
(73) Titulaire: Centre Scientifique et Technique du Batiment, 77420 Champs sur Marne (FR)
(72) Inventeur: Moularat, Stéphane, 77185 LOGNES (FR); Robine, Enric, 77400 LAGNY-SUR-MARNE (FR)
(74) Mandataire: Thomas, Nadine

(56) Documents cités:
- WO-A1-2008/125770
- Christoph-Bernward Brattig: "Produktion von leichtflüchtigen organischen Substanzen (MVOC) durch Schimmelpilze" In: "Produktion von leichtflüchtigen organischen Substanzen (MVOC) durch Schimmelpilze", 27 octobre 2010 (2010-10-27), Medizinische Fakultät Charité - Universitätsmedizin Berlin, Berlin, XP055021547, * page 55 - page 57 * * page 63 - page 68 * * page 71 - page 72 *
- K WILKINS: "Volatile metabolites from mold growth on building materials and synthetic media", CHEMOSPHERE, vol. 41, no. 3, 1 août 2000 (2000-08-01), pages 437-446, XP055021553, ISSN: 0045-6535, DOI: 10.1016/S0045-6535(99)00273-8

## Description

### DOMAINE TECHNIQUE DE L'INVENTION

La présente invention concerne un procédé de détermination d'une empreinte chimique spécifique de la production de mycotoxines dans des environnements intérieurs.

Par environnement intérieur on entend un espace confiné à l'intérieur d'un bâtiment qui est aéré de façon non continue. Des exemples d'environnements intérieurs peuvent être trouvés dans les habitations, les musées, les églises, les caves, les monuments historiques, les bâtiments administratifs, les écoles et les hôpitaux.

L'OMS, dans son rapport «WHO guidelines for indoor air quality : dampness and mould» (2009), rappelle l'importance sanitaire désormais reconnue des champignons microscopiques, colonisant nos habitats et notamment leur capacité à engendrer des pathologies toxiques liées aux mycotoxines qu'ils produisent. Aussi, la détection préventive de ces entités biologiques indésirables présente un intérêt croissant pour la protection des occupants.

Dans ce contexte, l'indice de contamination fongique mis au point par la demanderesse et décrit dans la demande WO 2008/125770 pourrait être complété pour une détection précoce de mycotoxines.

### ETAT DE LA TECHNIQUE ANTERIEURE

Ainsi, Zeringue et al. en 1993 ont constaté des différences d'émissions de COV entre des souches d'*Aspergillus flavus* toxinogènes (productrices d'aflatoxines) et des souches non toxinogènes.

En 1993, Desjardins détermine que le trichodiène volatil est le premier métabolite dans la voie de biosynthèse des trichothécènes. Ce résultat est également retrouvé par Jelen et al. (1995; 1997a; 1997b) qui établit une corrélation entre la synthèse de trichothécènes et la production de trichodiène et d'autres sesquiterpènes par *Fusarium sambucinum, F. sporotrichoides, F. poae et F. graminearum.*

Pasanen et al. (1996) indique que la production de terpènes et sesquiterpènes volatils par des souches de Fusarium est associée à la production de trichothécènes.

Concernant la production de mycotoxines, celle-ci n'est significativement décelable qu'après une contamination fongique avancée, alors que l'on souhaite éviter la génération de mycotoxines aéroportées. En outre une détection de mycotoxines s'avère très difficile en comparaison avec une détection de COV.

Pour pallier à ces inconvénients, on connaît de la demande WO 2008/125770 indiquée ci-dessus, un procédé de détection d'une contamination fongique d'un environnement intérieur à l'aide du calcul d'un indice chimique de contamination fongique. Cependant la réalisation de ce procédé ne permet pas spécifiquement de conclure s'il y a ou non production de mycotoxines.

### EXPOSE DE L'INVENTION

Dans ce contexte, il est particulièrement intéressant d'étudier la relation entre les émissions de COV et le caractère toxinogène d'une souche fongique.

La détermination d'une empreinte chimique spécifique de la production de mycotoxines permettrait alors de compléter les indices de contaminations fongiques déjà développés par la demanderesse, en fournissant des critères clairs et précis pour les décisions concernant l'occupation et la rénovation de bâtiments contaminés.

Ainsi, la demanderesse propose un procédé de détermination d'une production de mycotoxines dans un environnement intérieur comprenant les étapes :
(a) de prélèvement d'un échantillon d'air dans l'environnement intérieur, puis
(b) de détection de COV dans l'échantillon.
Selon un premier aspect, l'étape (b) comprend une recherche d'une empreinte chimique comprenant au moins une molécule cible qui est un COV, associé à une mycotoxicogénèse.

En particulier, la molécule cible est un COV cyclique, associé à une mycotoxicogénèse.

De façon particulièrement avantageuse, la détection de telles molécules cibles est plus facile et plus rapide que la détection de mycotoxines.

Ladite molécule cible est sélectionnée dans le groupe comprenant l'ylangène, le germacrène D, le 1-1-diméthylbutylbenzène, le 1,1,2-triméthyl-propyl-benzène, le 1-hexyltetradecyl-benzène, le tetratetracontane, le 1-éthyldecyl-benzène, le butylate d'hydroxytoluène, le 1-butyloctylbenzène, et le 1-propylnonylbenzène.

Selon une variante, l'empreinte chimique comprend au moins deux molécules cibles dont au moins une est une sesquiterpène.

De préférence, l'empreinte chimique comprend toutes lesdites molécules cibles.

Selon une variante intéressante, le procédé comprend une étape de recherche de zones de contamination fongique ; cette recherche sera réalisée avant le prélèvement d'échantillon d'air. Ainsi, dans le cas où un développement fongique est visible à l'oeil nu, et forme une zone de contamination fongique, on peut faire le prélèvement d'air en regard de cette zone de contamination. Une recherche de zones de contamination fongique peut aussi comprendre des analyses en microscopie, des tests microbiologiques ou biochimiques.

Selon un deuxième aspect, le procédé comprend les étapes:
(a) de prélèvement d'un échantillon d'air dans l'environnement intérieur ; puis
(b) de détection de COV dans l'échantillon, qui comprend une détection de la présence ou de l'absence de certains COV prédéterminés, issus du métabolisme fongique, ces COV prédéterminés comprenant au moins un COV de chacune des trois catégories de COV suivantes :
   (1) les COV qui sont émis indépendamment de l'espèce fongique et de son support et qui ne sont émis que par des espèces fongiques ;
   (2) les COV qui sont émis indépendamment de l'espèce fongique et du support, et qui sont émis par des espèces biologiques non fongiques ;
   (3) les COV qui sont émis en fonction de l'espèce fongique et/ou de son support;
(c) de calcul d'un indice chimique de contamination fongique en fonction respectivement de la présence et de l'absence des COV prédéfinis issus du métabolisme fongique.

Par « support » d'une espèce fongique, on entend le matériau sur lequel l'espèce fongique se développe, de préférence un matériau de construction tel que du papier peint, de la toile de verre ou autre.

Ensuite, le procédé comprend une étape (d) de recherche d'une empreinte chimique qui comprend au moins une molécule cible qui est un COV, associé à une mycotoxicogénèse.

Le procédé selon le deuxième aspect de l'invention est particulièrement utile pour la détection précoce d'une production de mycotoxines, c'est-à-dire avant l'apparition de quantités détectables de mycotoxines. Cette possibilité de détection précoce est d'autant plus intéressante qu'elle ne nécessite pas une détection directe de mycotoxines. On peut ainsi conclure à la production de mycotoxines à un stade précoce de développement des champignons. Par « stade précoce » de développement, on entend un stade où les champignons sont invisibles à la surface du support, et de préférence indécelables par l'analyse microbiologique de l'air, mais produisent néanmoins des métabolites et produits de dégradation inhalables et responsables dans certains cas de maladies.

### DESCRIPTION DETAILLEE D'UN MODE DE REALISATION

Une étude des émissions de COV issus d'une souche d'*Aspergillus versicolor* a été effectuée dans différentes conditions de croissance :
- des conditions de croissance permettant la production de stérigmatocystine, dites conditions de mycotoxicogénèse, et
- des conditions de croissance ne permettant pas de production de stérigmatocystine, dites conditions de non mycotoxicogénèse.

Des échantillons d'*Aspergillus versicolor* ont été mis en culture sur une toile de verre et sur du papier peint. La culture a été effectuée sur un milieu nutritif dont la composition est la suivante ; pour 1 litre de solution tamponée en pH (pH 7,4). :

| | |
|---|---|
| K2HPO4 : 1 g | FeSO4; 7H2O : 0,01 g |
| KCl : 0,5 g | Glucose : 31,5 g |
| MgSO4; 7H2O : 0,5 g | NaNO3 : 3,5.10-2 g |

La solution tamponnée à pH 7,4 est par exemple préparée avec 250 ml de KH2PO4 à 0,1 M, auxquels on ajoute 145,5 ml de NaOH à 0,1 M ; et la solution est complétée à 500 ml avec de l'eau distillée.

Après une culture dans des conditions de mycotoxicogénèse, des prélèvements d'air ont été effectués dans les deux cas. Aucune production de mycotoxine (notamment stérigmatocystine) n'a été détectée sur les essais sur le support en toile de verre.

En revanche, cette étude a montré que sur le support en papier peint, il y a non seulement une production de mycotoxines mais aussi une production de plus d'une dizaine de composés (dont des sesquiterpènes) en lien avec les schémas métaboliques complexes empruntés par la mycotoxicogénèse. Ces composés peuvent donc être utilisés comme molécules cibles. Des analyses complémentaires du support papier peint ont révélé que ces composés ne proviennent pas du papier peint. Ceci démontre que ces composés sont directement liés à la production de mycotoxines.

En particulier les molécules cibles suivantes ont été identifiées :
cububène, cadiène, copaène, ylangène, germacrène D, muurolane, 1-1-diméthylbutylbenzène, 1,1,2-triméthyl-propyl-benzène, 1-hexyltetradecyl-benzène, tetratetracontane, 1-éthyldecyl-benzène, butylate d'hydroxytoluène, 1-butyloctylbenzène, 1-propylnonylbenzène, 2-méthylisobornéol, ainsi que des sesquiterpènes.

D'autres molécules cibles peuvent être identifiées. De manière générale, des molécules cibles de ce type peuvent consister en tout composé en lien avec les schémas métaboliques empruntés par la mycotoxicogénèse, c'est-à-dire un composé produit par les champignons pendant la mycotoxicogénèse. Il ressort des premières analyses qu'une forte proportion de ces composés comporte des cycles (parfois benzeniques). En outre, ces composés ont, pour la plupart, une masse moléculaire supérieure à 204.

Ainsi, on peut déterminer une empreinte chimique spécifique de la production de mycotoxines permettant de compléter les indices de contamination fongique déjà développés dans la demande WO 2008/125770, en fournissant des critères clairs et fiables pour les décisions concernant par exemple l'occupation et la rénovation de bâtiments contaminés.

Dans le cas d'*Aspergillus versicolor,* une empreinte chimique observée est constituée par les molécules cibles suivantes : cububène, cadiène, copaène, ylangène, germacrène D, muurolane, 1-1-diméthylbutylbenzène, 1,1,2-triméthyl-propyl-benzène, 1-hexyltetradecyl-benzène, tetratetracontane, 1-éthyldecyl-benzène, butylate d'hydroxytotuène, 1-butyloctylbenzène, 1-propylnonylbenzène, 2-méthylisobornéol, ainsi que des sesquiterpènes.

D'un point de vue pratique, après détermination de la présence d'un développement fongique par l'indice de contamination fongique, la recherche des cibles spécifiques de la mycotoxicogénèse permet d'alerter sur une production probable de mycotoxines associée à ce développement fongique. Le nombre de cibles présentes est alors lié à une probabilité d'exposition à ces métabolites.

Dans la mise en oeuvre du procédé selon une variante préférée, on réalise, successivement les étapes de :
a) prélèvement d'un échantillon d'air dans un environnement intérieur, par exemple à proximité de zones suspectées d'êtres contaminées ;
b) détection de COV dans l'échantillon, qui comprend une détection de la présence ou de l'absence de certains COV prédéterminés issus du métabolisme fongique, ces COV prédéterminés comprenant au moins un COV de chacune des trois catégories de COV suivantes :
   (1) les COV qui sont émis indépendamment de l'espèce fongique et de son support et qui ne sont émis que par des espèces fongiques ;
   (2) les COV qui sont émis indépendamment de l'espèce fongique et du support, mais qui peuvent également avoir d'autres origines biologiques par COV ayant « d'autres origines biologiques », on entend notamment des COV émis par des espèces biologiques non fongiques ;
   (3) les COV qui sont émis en fonction de l'espèce fongique et/ou de son support
c) calcul d'un indice chimique de contamination fongique en fonction respectivement de la présence et de l'absence des COV prédéfinis, issus du métabolisme fongique, conformément au procédé décrit dans la demande WO 2008/125770, pour déterminer s'il y a une contamination fongique ;
   Ensuite, pour déterminer s'il y'a une production de mycotoxines, on réalise en outre les étapes de :
d) recherche d'au moins une molécule cible, issue d'une mycotoxicogénèse, dont la masse moléculaire est optionnellement supérieure à 200g/mol, en particulier au moins une molécule cible sélectionnée dans le groupe comprenant le cububène, le cadiène, le copaène, l'ylangène, le germacrène D, le muurolane, le 1-1-diméthylbutylbenzène, le 1,1,2-triméthyl-propyl-benzène, le 1-hexyltetradecyl-benzène, le tetratetracontane, le 1-éthyldecyl-benzène, le butylate d'hydroxytoluène, le 1-butyloctylbenzène, le 1-propylnonylbenzène, le 2-méthylisobornéol, au moins une sesquiterpene ;
e) recherche d'une empreinte chimique comprenant au moins deux desdites molécules cibles.

De façon intéressante, nouvelle et inventive, les résultats issus des étapes d) et e) permettent de déterminer avec précision, clarté et fiabilité s'il y a ou non une production de mycotoxines.

Ce mode de réalisation aboutit bien entendu à des résultats plus complets que ceux de l'art antérieur, en ce qu'on arrive non seulement à conclure à une contamination fongique sans aucun signe visible de développement fongique, mais en plus on arrive à déterminer de manière précise et fiable qu'il y a une production de mycotoxines.

Dans une autre variante de l'invention, on peut aussi rechercher des zones de contamination fongique ; puis prélever un échantillon d'air à proximité de ces zones de contamination fongique avant de rechercher ladite ou desdites molécules cibles évoquées ci-dessus.

Une telle recherche de zones de contamination fongique peut se faire par exemple à l'oeil nu, par des analyses en microscopie ou par des tests microbiologiques ou biochimiques.

Le prélèvement de l'échantillon d'air est par exemple réalisé par échantillonnage diffusif sur un adsorbant solide de type carbographe 4. La détection est par exemple réalisée par chromatographie en phase gazeuse suivie de spectrométrie de masse (GC/MS). D'autres méthodes de détection peuvent être utilisées.

## Revendications

1. Procédé de détermination d'une production de mycotoxines dans un environnement intérieur comprenant les étapes de :
(a) prélèvement d'un échantillon d'air dans l'environnement intérieur, puis
(b) détection de COV dans l'échantillon,
**caractérisé en ce que** l'étape (b) comprend une recherche d'une empreinte chimique comprenant au moins une molécule qui est un COV, associé à une mycotoxicogénèse, sélectionnée dans le groupe comprenant l'ylangène, le germacrène D, le 1-1-diméthylbutylbenzène, le 1,1,2-triméthyl-propyl-benzène, le 1-hexyltetradecyl-benzène, le tetratetracontane, le 1-éthyldecyl-benzène, le butylate d'hydroxytoluène, le 1-butyloctylbenzène et le 1-propylnonylbenzène.

2. Procédé selon la revendication 1, **caractérisé en ce que** ladite empreinte chimique comprend au moins deux molécules cibles sélectionnées dans le groupe comprenant le cububène, le cadiène, le copaène, l'ylangène, le germacrène D, le muurolane, le. 1-1-diméthylbutylbenzène, le 1,1,2-triméthyl-propyl-benzène, le 1-hexyltetradecyl-benzène, le tetratetracontane, le 1-éthyldecyl-benzène, le butylate d'hydroxytoluène, le 1-butyloctylbenzène, le 1-propylnonylbenzène, le 2-méthylisobornéol, au moins une sesquiterpene, et dont au moins une molécule cible est une sesquiterpène.

3. Procédé selon l'une des revendications 1 ou 2, **caractérisé en ce que** ladite empreinte chimique comprend toutes lesdites molécules cibles.

4. Procédé selon l'une des revendications 1 à 3, comprenant une étape de recherche de zones de contamination fongique réalisée avant l'étape (a).

5. Procédé de détermination d'une production de mycotoxines dans un environnement intérieur selon la revendication 1, comprenant les étapes de :
(a) prélèvement d'un échantillon d'air dans l'environnement intérieur ; puis
(b) détection de COV dans l'échantillon, qui comprend une détection de la présence ou de l'absence de certains COV prédéterminés issus du métabolisme fongique, ces COV prédéterminés comprenant au moins un COV de chacune des trois catégories de COV suivantes :
(1) les COV qui sont émis indépendamment de l'espèce fongique et de son support et qui ne sont émis que par des espèces fongiques ;
(2) les COV qui sont émis indépendamment de l'espèce fongique et du support, et qui sont émis par des espèces biologiques non fongiques;
(3) les COV qui sont émis en fonction de l'espèce fongique et/ou de son support ;
(c) calcul d'un indice chimique de contamination fongique en fonction respectivement de la présence et de l'absence des COV prédéfinis issus du métabolisme fongique,
**caractérisé en ce que** le procédé comprend ensuite une étape (d) de recherche d'une empreinte chimique comprenant au moins une molécule cible qui est un COV, associé à une mycotoxicogénèse.

6. Procédé selon la revendication 5, **caractérisé en ce que** ladite molécule cible est sélectionnée dans le groupe comprenant le cububène, le cadiène, le copaène, l'ylangène, le germacrène D, le muurolane, le 1-1-diméthylbutylbenzène, le 1,1,2-triméthyl-propyl-benzène, le 1-hexyltetradecyl-benzène, le tetratetracontane, le 1-éthyldecyl-benzène, le butylate d'hydroxytoluène, le 1-butyloctylbenzène, le 1-propylnonylbenzène, le 2-méthylisobornéol, au moins une sesquiterpène.

7. Procédé selon l'une des revendications 5 ou 6, **caractérisé en ce que** l'empreinte chimique comprend au moins deux molécules cibles dont au moins une est une sesquiterpène.

8. Procédé selon l'une des revendications 5 à 7, **caractérisé en ce que** l'empreinte chimique comprend toutes lesdites molécules cibles.

## Patentansprüche

1. Bestimmungsverfahren einer Mycotoxin-Produktion in einem Innenumfeld, umfassend die Stufen:
(a) Entnahme einer Luftprobe im Innenumfeld, dann
(b) Erfassung von VOC in der Probe,
**dadurch gekennzeichnet, dass** die Stufe (b) eine Suche eines chemischen Fingerabdrucks umfasst, umfassend wenigstens ein Molekül, das ein VOC ist, das einer Mycotoxicogenese zugeordnet ist, die in einer Gruppe ausgewählt ist, umfassend Ylangen, Germacren D, 1-1-Dimethylbutylbenzol, 1, 1, 2-Trimethyl-Propyl-Benzol, 1-Hexyltetradecyl-Benzol, Tetrateracontan, 1-Ethyldecyl-Benzol, Hydroxytoluol-Butylat, 1- Butyloctylbenzol und 1-Propylnonylbenzol.

2. Verfahren gemäß Anspruch 1, **dadurch gekennzeichnet, dass** der chemische Fingerabdruck wenigstens zwei Zielmoleküle umfasst, die aus der Gruppe ausgewählt sind, umfassend Kubeben, Kadinen, Copaen, Ylangen, Germacren D, Muurolan, 1-1-Dimethylbutylbenzol, 1, 1, 2-Trimethylpropyl-Benzol, 1-Hexyltetradecyl-Benzol, Tetratetracontan, 1-Ethyldecyl-Benzol, Hydroxytoluol-Butylat, 1-Butyloctylbenzol, 1-Propylnonylbenzol, 2-Methylisoborneol, wenigstens ein Sesquiterpen und von dem wenigstens ein Zielmolekül ein Sesquiterpen ist.

3. Verfahren gemäß Anspruch 1 oder 2, **dadurch gekennzeichnet, dass** der chemische Fingerabdruck alle die genannten Zielmoleküle umfasst.

4. Verfahren gemäß Anspruch 1 bis 3, umfassend eine Suchstufe von Schimmelkontaminationsbereichen, die vor der Stufe (a) realisiert wird.

5. Bestimmungsverfahren einer Mycotoxin-Produktion in einem Innenumfeld gemäß Anspruch 1, umfassend die Stufen:
(a) Entnahme einer Luftprobe im Innenumfeld; dann
(b) Erfassung von VOC in der Probe, die eine Erfassung des Vorhandenseins oder des Fehlens von bestimmten, vorbestimmten VOC umfasst, welche aus dem Schimmelmetabolismus hervorgegangen sind, wobei diese vorbestimmten VOC wenigstens ein VOC jedes der drei folgenden VOC-Kategorien umfasst:
(1) die VOC, die unabhängig von der Schimmelart und seinem Träger ausgegeben werden und die nur von Schimmelarten ausgegeben werden;
(2) die VOC, die unabhängig von der Schimmelart und dem Träger ausgegeben werden und die von biologischen, schimmelfremden Arten ausgegeben werden;
(3) die VOC, die in Abhängigkeit von der Schimmelart und/ oder seinem Träger ausgegeben werden;
(c) Berechnung eines chemischen Schimmelkontaminationsindexes in Abhängigkeit jeweils von dem Vorhandensein und dem Fehlen der vordefinierten, aus dem Schimmelmetabolismus hervorgegangenen VOC,
**dadurch gekennzeichnet, dass** das Verfahren anschließend eine Suchstufe (d) eines chemischen Fingerabdrucks umfasst, umfassend wenigstens ein Zielmolekül, das ein VOC ist, das einer Mycotoxicogenese zugeordnet ist.

6. Verfahren gemäß Anspruch 5, **dadurch gekennzeichnet, dass** das genannte Zielmolekül ausgewählt ist in der Gruppe umfassend Kubeben, Kadinen, Copaen, Ylangen, Germacren D, Muurolan, 1-1-Dimethylbutylbenzol, 1, 1, 2-Trimethylpropyl-Benzol, 1-Hexyltetradecyl-Benzol, Tetratetracontan, 1-Ethyldecyl-Benzol, Hydroxytoluol-Butylat, 1-Butyloctylbenzol, 1-Propylnonylbenzol, 2-Methylisoborneol, wenigstens ein Sesquiterpen.

7. Verfahren gemäß Anspruch 5 oder 6, **dadurch gekennzeichnet, dass** der chemische Fingerabdruck wenigstens zwei Zielmoleküle umfasst, von dem wenigstens eines ein Sesquiterpen ist.

8. Verfahren gemäß Anspruch 5 bis 7, **dadurch gekennzeichnet, dass** der chemische Fingerabdruck alle die genannten Zielmoleküle umfasst.

## Claims

1. Process for determining mycotoxin production in an interior environment comprising the steps of:
a) collecting an air sample from the interior environment, and then
b) detecting VOCs in the sample,
**characterized in that** step b) includes a search for a chemical fingerprint comprising at least one target molecule which is a VOC associated with mycotoxin production, selected from the group comprising ylangene, D germacrene, 1,1-dimethylbutylbenzene, 1,1,2-trimethyl-propyl-benzene, 1-hexyltetradecylbenzene, tetratetracontane, 1-ethyldecylbenzene, hydroxytoluene butylate, 1-butyloctylbenzene and 1-propylnonylbenzene.

2. Process according to claim 1, **characterized in that** said target molecule comprises at least two target molecules which are selected from the group comprising cububene, cadiene, copaene, ylangene, D germacrene, muurolane, 1,1-dimethylbutylbenzene, 1,1,2-trimethyl-propyl-benzene, 1-hexyltetradecylbenzene, tetratetracontane, 1-ethyldecylbenzene, hydroxytoluene butylate, 1-butyloctylbenzene, 1-propylnonylbenzene, 2-methylisoborneol, at least one sesquiterpenes, and of which at least one target molecule is a sesquiterpene.

3. Process according to any one of claims 1 or 2, **characterized in that** the chemical fingerprint includes all said target molecules.

4. Process according to any one of claims 1 to 3, **characterized in that** it includes a step of searching for fungal contamination zones conducted before step (a).

5. Process for determining mycotoxin production in an interior environment according to claim 1 comprising the steps of:
a) Collecting an air sample from the interior environment; then
b) Detecting VOCs in the sample, which includes detection of the presence or absence of certain set VOCs coming from fungal metabolism where these set VOCs include at least one VOC from each of the following three categories of VOCs:
1) VOCs that are released independently of the fungal species and the medium thereof and that are released only by fungal species;
2) VOCs that are released independently of the fungal species and the medium, and which are released by non-fungal biological species;
3) VOCs that are released as a function of the fungal species and/or the medium thereof;
c) Calculating a fungal contamination chemical index as a function respectively of the presence and absence of the set VOCs coming from fungal metabolism, **characterized in that** the process includes a step (d) of searching for a chemical fingerprint which comprises at least one target molecule which is a VOC associated with mycotoxin production.

6. Process according to claim 5, **characterized in that** said target molecule is selected from the group comprising cububene, cadiene, copaene, ylangene, D germacrene, muurolane, 1,1-dimethylbutylbenzene, 1,1,2-trimethyl-propyl-benzene, 1-hexyltetradecylbenzene, tetratetracontane, 1-ethyldecylbenzene, hydroxytoluene butylate, 1-butyloctylbenzene, 1-propylnonylbenzene, 2-methylisoborneol and at least one sesquiterpene.

7. Process according to any one of claims 5 or 6, **characterized in that** the chemical fingerprint comprises at least two target molecules of which at least one is a sesquiterpene.

8. Process according to any one of claims 5 to 7, **characterized in that** the chemical fingerprint comprises all said target molecules.
